(19) Europäisches Patentamt
European Patent Office
Office européen des brevets

(11) Publication number: 0 038 572
B1

(12) EUROPEAN PATENT SPECIFICATION

(45) Date of publication of patent specification: 21.03.84

(51) Int. Cl.[3]: C 07 D 219/10, A 61 K 31/435

(21) Application number: 81103067.5

(22) Date of filing: 23.04.81

# (54) 1-Nitro-9-hydroxyalkylaminoacridines or their salts.

(30) Priority: 23.04.80 PL 223740

(43) Date of publication of application:
28.10.81 Bulletin 81/43

(45) Publication of the grant of the patent:
21.03.84 Bulletin 84/12

(84) Designated Contracting States:
BE CH DE FR GB LI SE

(56) References cited:
GB - A - 1 528 723
NL - C - 10 194
US - A - 4 150 231

CHEMICAL ABSTRACTS, vol. 74, no. 23, 7th June 1971, page 457, no. 125479x Columbus, Ohio, U.S.A:

The file contains technical information submitted after the application was filed and not included in this specification

(73) Proprietor: Politechnika Gdanska
ul. Majakowskiego 11/12
Gdansk (PL)

(72) Inventor: Wysocka-Skrzela, Barbara
ul. Szeroka 15/4
Gdansk (PL)
Inventor: Ledóchowski, Andrzej
ul. Subieslawa 27A/27
Gdansk-Oliwa (PL)
Inventor: Radzikowski, Czeslaw
ul. Lwowska 47/35
Wroclaw (PL)

(74) Representative: Hansen, Bernd, Dr.rer.nat. et al,
Hoffmann, Eitle & Partner Patentanwälte
Arabellastrasse 4
D-8000 München 81 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 038 572 B1

1-Nitro-9-hydroxyalkylaminoacridines or their salts

The subject of the present invention are 1-nitro-9-hydroxyalkylaminoacridines or their salts of the formula 1:

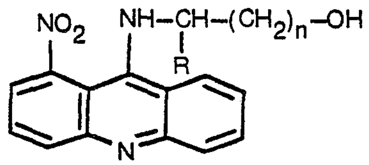

wherein R is hydrogen, and n = 1, 2 or 3; or R is ethyl, and n = 1.

These compounds are useful as anti-neoplastic agents. Until now from British Patent No. 1,093,847 1-nitro-9-dialkylaminoalkylaminoacridines are known, characterized by formula 2:

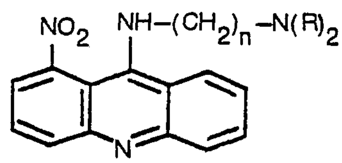

wherein R is methyl or ethyl, n is 2 or 3.

All of these compounds have two identical substituents at the terminal nitrogen ($N^{\omega}$) in the amino-alkylamino chain at a position of the acridine system.

1-nitro-9-alkylaminoalkylaminoacridines of formula 3

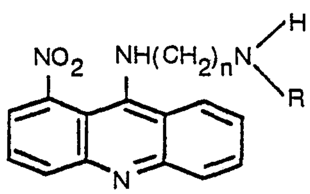

in which R is a methyl, ethyl, propyl, isopropyl, butyl, isopentyl, benzyl or cyclohexyl group and n is 2 or 3, are disclosed in British Patent No. 1,528,723.

Compounds having the formula 4

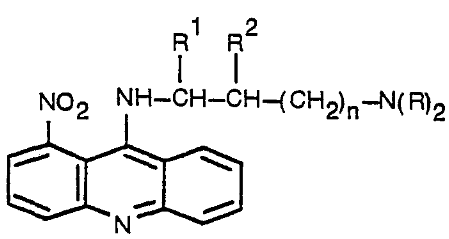

in which R is a lower alkyl such as methyl or ethyl, $R^1$ is a hydrogen atom or methyl, $R^2$ is the same as $R^1$, with the proviso that $R^1$ is not equal to $R^2$ and n = 0 or 2 are disclosed in U.S. patent 4,150,231.

A disadvantage of the compounds described above is their high toxicity, instability, especially in aqueous solutions, in which they are easily hydrolysed to the 1-nitroacridone, which is a water insoluble product without biologic activities. Aqueous solutions of these compounds have a lower pH (pH about 4) which is responsible for side effects such as a local irritating action on intravenous injection.

Moreover, they exerted unpleasant effects on the digestion track, e.g. long-lasting nausea and vomiting.

These effects very frequently render their use to be impossible or limit their use and restrict the preparation of various medicinal forms thus decreasing the range of their application.

The invention relates to 1-nitro-9-hydroxyalkylaminoacridines or salts thereof having the general formula 1, where R is H, n = 1, 2 or 3, or R is ethyl and n = 1.

The subject compounds differ from those described in the previous patents by substituent at

position 9 of the acridine system. Contrary to the derivatives described above, these compounds have a hydroxyalkylamine group at this position.

1-nitro-9-hydroxyalkylaminoacridines have high anti-neoplastic activity, which was confirmed by many tests in vitro and in vivo. The pharmacological examination showed, they are less toxic than the derivatives of acridines known from GB—PS 1 093 847, 1 528 723 and US—PS 4,150,231. One of the compounds according to the invention, i.e. compound C—857, has been qualified for clinical evaluations after extensive pharmacological studies.

The anti-neoplastic properties of the subject group of new derivatives of 1-nitro-9-hydroxyalkylaminoacridines were investigated many times, using the tests described below.

I. Methods *in vitro*

*1. Tissue culture (LS-leucocytic series line)*

The investigations were carried out using the tissue culture method elaborated by Eagle and Faley. Cancer Research *18*, 1017 (1958), modified by Smith and colaborators (Cancer Research *29*, 843 (1959)).

The experiments were carried out in neoplastic cells of human origin, so-called LS-lines (leucocytic series lines) using the Eagle's nutrient medium with 1% of calf's serum added.

The tests were performed in test-tubes, inoculating with 4 ml of the suspension (40—80 thousand cells), which is equivalent to 40—80 $\mu$g of cell protein.

The growth of the culture was determined by estimating the increase in cell protein. This estimation was carried out photometrically, using Folin-Cicalteau's reagent according to the method elaborated by Oyama (Proc. Soc. Exp. Biol. Med. *91*, 305 (1956)).

Simultaneously with inoculation of test-tubes with cells, the amount of 0,2 ml of aqueous solution of the compound being tested was added, so that the concentration should be obtained as follows: 100, 10, 1, 0.1, 0.01 and 0.001 $\mu$g/ml of the nutrient medium. The test-tubes were incubated at a temperature of 37°C. After 72 hours the increase in cytoprotein in the test-tubes was determined. Each concentration was carried out in duplicate. The concentration of the substance at which an inhibition of the increase in cell protein of 50% was obtained compared with the control and the so-called $ED_{50}$ (or $ID_{50}$) was also determined. The percent of this inhibition was calculated according to the following formula:

$$\%\ \text{inhibition} = \frac{\text{final protein concentration in control} - \text{final protein concentration in test}}{\text{final protein concentration in control} - \text{initial protein concentration in control}} \times 100$$

According to the generally accepted norms, commonly accepted for active compounds there are admitted such compounds whose $ED_{50}$ value is equal to 1 $\mu$g/ml. Leiter and collaborators (Cancer Res. Supl. Cancer Chemoth. Screening Data XXXV, 2, *25*, 522, (1965)) are of the opinion, that such compounds should undergo clinical investigation paying no regard to results obtained during the investigation "in vivo".

The compounds belonging to the group mentioned above were tested by this method several times.

*2. The Miyamura Test (cells of the Ehrlich cancer)*

The method involves the determination of dehydrogenase inhibition activity Ehrlich cancer cells ($5 \times 10^6$ in ml) by the compound under test, which is expressed as the diameter of the zone of nonreduced redox-dye (resazurine), which is formed around a cylinder containing 1% of solution of the compound tested after 5 hours of incubation at a temperature of 37°C.

According to commonly accepted criteria, the compounds for which the inhibition zone is at least 20 mm, are accepted as active.

The acridines of the formula 1 show exceptionally high antineoplastic activity, which amounts from 31—33 mm for several compounds by this test.

*3. Inhibition of germination of the cress seeds (growth test) (Lepidium sativum)*

On a Petri dish 80 mm in diameter 20—25 cress seeds are placed, as uniformly as possible, on two layers of filter-paper.

Subsequently 30 ml of a solution of the compound tested with a concentration of 1 mg/ml are poured onto these dishes and distilled water is added to the control dish. The dishes were incubated for 24 hours at a temperature of 20—30°C and then the length of the growing shoots was measured.

The effect of the inhibition is expressed in percent of the reduction of the average length of growing shoots tested, in comparison to the control.

The percent of germination inhibition of cress seeds for the applied group of compounds is 87—94%.

II. Methods *in vivo*

*The growth inhibition of Crocker sarcoma (Sa-180) in mice.*

For these experiments three-months old mice of about 25 g in weight were used. Each was inoculated with mouse sarcoma (Sa-180) and then they were divided into groups: a group of 10 control mice, and two to four test groups of 7 mice each.

The compounds to be investigated were administered intraperitoneally in appropriate doses after previous determination of maximum tolerance dose.

The percent difference between the mean weight of tumors isolated from control and test mice was accepted as a criterion for the evaluation of antineoplastic activity of the compounds, taking into account the toxic effects. Compounds were regarded as active if they exerted at least 40% tumor growth inhibition in at least 2 tests without deaths of animals (less than 2 mice) and showed mean weight losses no higher than 4 g.

The *in vivo* investigations of several compounds of the present invention were carried out repeatedly, e.g. for the preparation allocated the code number C—857 i.e. 1-nitro-9-(hydroxyethyl-amino)acridine hydrochloride, 33 determinations in ten series were accomplished using different doses and an optimal growth inhibition of the Crocker sarcoma (Sa-180) was shown to depend upon the dose.

After doses of between 0.2—0.8 mg/kg the percent of inhibition was 44—86% respectively. Similarly, for the preparation allocated the code number C—934, i.e. 1-nitro-9-(hydroxybutylamino)-acridine hydrochloride, after doses of between 0.2—0.4 mg/kg percent of inhibition was 45—60% respectively.

*Pharmacological examination*

Pharmacological examination of the compound C—857 was carried out in mice, rats and rabbits. The acute, subacute, and long-term toxicity were studied in rats and rabbits (orally and intraperitoneally) and in mice (intraperitoneally). $LD_{50}$ is 10.58 mg/kg in mice (intraperitoneally), 9.2 mg/kg in rats (intravenously), and 11.4 mg/kg in rabbits. In the subacute toxicity studies (3 weeks) 1/60 and 1/120 of $LD_{50}$ were considered; in the long-term toxicity evaluations (3 months) — 1/200 and 1/400 of $LD_{50}$.

The maximal tolerated dose, $LD_{10}$, was 2.5 mg/kg in mice when compound C-857 was administered intraperitoneally. When administered intravenously, however, in doses increasing to 20 mg/kg preparation C-857 did not exert any significant effect on the circulatory system and showed only a weak spasmolytic action. Its effect on central nervous system was weak and irregular. In a dose corresponding to 1/2 of $LD_{50}$ it inhibits the spontaneous mobility and shortens hexobarbital anaesthesia. It does not show any analgesic or anti-convulsant activity. Its immunosuppressive action measured in contact allergy tests, local transplant-host reaction tests and post adjuvant multiarticular inflammation is minimal and appears only after the total dose corresponding to 1/5 of $LD_{50}$.

The following effects were observed in the subacute toxicity investigations: moult, dyspnea, lack of thirst, diarrhoea, and loss of body weight. About 15—40% of total number of mice died after a dose of 1/120 of $LD_{50}$. The increase in the number of leucocytes (particularly in rabbits) and decrease in relative number of lymphocytes were observed. However, no changes in the number of red cells were found. Liver and kidney function tests showed no pathological abnormalities. The preparation C-857 induced some drop in the urine level in rabbits. The histologic studies were limited to necrotic inflammation of the liver capsule and peritoneum of intestine wall. No pathological abnormalities in internal organs (liver, kidney, thymus gland) were observed.

Long-term toxicity observation showed only general changes involving decrease in physical activity, lack of third or occasional diarrhoea. The preparation C-857 administerred intraperitoneally exerted locally irritating action. Insignificant changes in liver and kidneys were reported, though no changes were found in thymus gland and intestine. The preparation C-857 was found not to change the spleen-liver index.

Hemotologic studies showed a decrease in the value of hematocrit (in rabbits), leucocytosis and decrease of relative content of cosinophils (in rabbits and rats). The evaluation of activity of AspAT and ALAT enzymes revealed no changes. Slight changes, however, were observed in activity of alkaline phosphatase. No changes of urea and creatinine content were found.

The preparation C-857 was found not to produce myelotoxic effect on cells forming colonies in marrow in mice, splenic colony technique, even in a dose equal to $LD_{50}$. The effect of action of compound C-857 is similar to that of Bleomicine which is non-toxic for marrow.

The preparation C-857 did not exert vomiting effect in pigeons.

The 1-nitro-9-hydroxyalkylaminoacridines and the method for obtaining them, are illustrated by the following Examples.

Example 1

To 6.4 g of 1-nitro-9-phenoxyacridine dissolved in 20 g of freshly distilled phenol, 2 g of 2-aminoethanol hydrochloride was added and the mixture heated for 40 minutes at a temperature of 80°C. The reaction mixture was then cooled, diluted with ether and then poured into a dry ether which was

acidified with an ethereal solution of hydrogen chloride. The orange-coloured precipitate of 1-nitro-9-(2-hydroxyethylamino)-acridine hydrochloride, obtained in this way was filtered and crystallized from dry ethanol. The melting point of the compounds obtained was 170°C, with decomposition. Yield 91%.

*Chromatographic analysis:*
1) on neutral aluminium oxide 60F—254 (Merck) in solvent system benzene : acetone : methanol (3:1:1), $R_F = 0.6$
2) on the silica gel (Merck) in solvent system butanol : acetic acid : water (4:1:1), $R_F = 0.45$

*Elementary analysis for the formula:*
$C_{15}H_{14}N_3O_3Cl$:

| | | | |
|---|---|---|---|
| calculated: | 56.47% C, | 4.42% H, | 13.17% N |
| determined: | 56.44% C, | 4.40% H, | 13.03% N |

Example 2

3.16 g of 1-nitro-9-phenoxyacridine was dissolved in 15 g of phenol and 1 g of 3-aminopropanol hydrochloride was added and heated 30 minutes at a temperature of 60°C. After heating, the reaction mixture was cooled, diluted with benzene and poured into anhydrous benzene. The crude yellow solid was precipitated, washed with ether and recrystallized from a mixture ethanol/ether. Obtained in this way 1-nitro-9(3-hydroxy propylamino)-acridine hydrochloride have the melting point 180°C with decomposition.

*Chromatographic analysis:*
1) on neutral aluminium oxide (Merck) in the solvent system: benzene : acetone : methanol (3:1:1), $R_F = 0.55$
2) on the silica gel (Merck) in the solvent system: butanol : acetic acid : water (4:1:1), $R_F = 0.45$

*Elementary analysis for the formula:*
$C_{16}H_{16}O_2N_3Cl$:

| | | | |
|---|---|---|---|
| calculated: | 57.53% C, | 4.8% H, | 12.58% N |
| found: | 57.63% C, | 4.97% H, | 12.43% N |

Example 3

3.9 g of 1-nitro-9-phenoxyacridine was dissolved in 20 ccm of phenol, 1.1 ccm of 2-amino-1-butanol was added and heated for 20 minutes at a temperature of 90°C. The reaction mixture was then poured into anhydrous ether. After distilling off part of the solvent used the crude-oil product obtained was crystallized from a mixture of benzene and petroleum ether; melting point 107°C, yield, 99%. Orange-yellow base of 1-nitro-9(N-2-ethyl-butanol-1)-acridine obtained in this way may be converted into its corresponding salts of organic and mineral acids, i.e. hydrobromide acid, citric acid, tertaric acid, methane sulphonic acid, lactic acid.

*Chromatographic analysis:*
1) on neutral aluminium oxide (Merck) in the solvent system: benzene : acetone : methanol (3:1:1), $R_F = 0.7$
2) on the silica gel (Merck) in solvent system: butanol : acetic acid : water (4:1:1), $R_F = 0.6$

*Elementary analysis for the formula:*
$C_{17}H_{18}O_3N_3Cl$ (for the hydrochloride salt)

| | | | |
|---|---|---|---|
| calculated: | 58.67% C, | 5.21% H, | 12.08% N |
| found: | 58.80% C, | 5.17% H, | 11.84% N |

Example 4

To 6.4 g of 1-nitro-9-phenoxyacridine which was dissolved in 30 ccm of phenol, 2.4 g 4-amino-1-butanol hydrochloride was added. The reaction mixture was heated for 20 minutes at a temperature of 100°C. After cooling, the reaction mxiture was poured into 1.5 l of cooled anhydrous ether. The orange precipitate of 1-nitro-9(4-hydroxybutylamino)acridine hydrochloride was filtered and crystallized from a mixture of anhydrous methanol-ether. The melting point after crystallization was 168°C with decomposition: yield 90%.

*Chromatographic analysis:*
1) on neutral aluminium oxide (Merck) in the solvent system: benzene : acetone : methanol (3:1:1), $R_F = 0.65$
2) on the silica gel (Merck) in solvent system: butanol : acetic acid : water (4:1:1), $R_F = 0.5$

*Elementary analysis for the formula:*
$C_{17}H_{18}O_3N_3Cl$

| | | | |
|---|---|---|---|
| calculated: | 58.67% C, | 5.21% H, | 12.08% N |
| found: | 58.69% C, | 5.20% H, | 12.16% N |

**Claim**

1-Nitro-9-hydroxyalkylaminoacridines or their salts of the formula 1

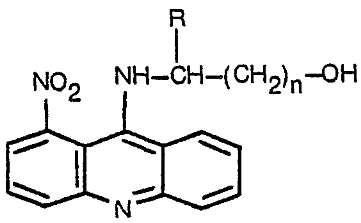

wherein R is H and n = 1, 2 or 3; or R is ethyl and n = 1.

**Patentanspruch**

1-Nitro-9-hydroxyalkylaminoacridine oder deren Salze entsprechend der Formel 1

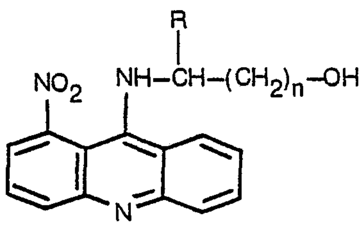

worin R Wasserstoff darstellt und n = 1, 2 oder 3; oder R Ethyl bedeutet und n = 1.

**Revendication**

1-nitro-9-hydroxyalkylaminoacridines ou leurs sels de formule 1

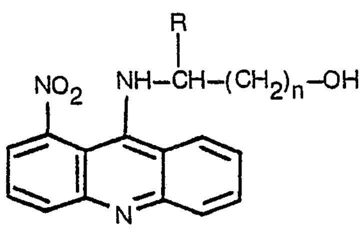

dans laquelle R représente H et n = 1, 2 ou 3, ou bien R représente le groupe éthyle et n = 1.

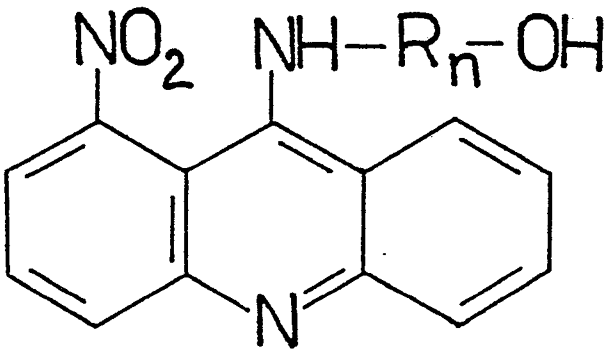

FORMULA 1

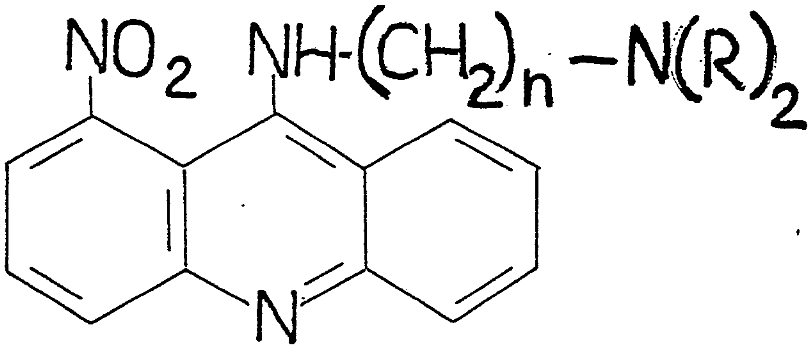

FORMULA 2

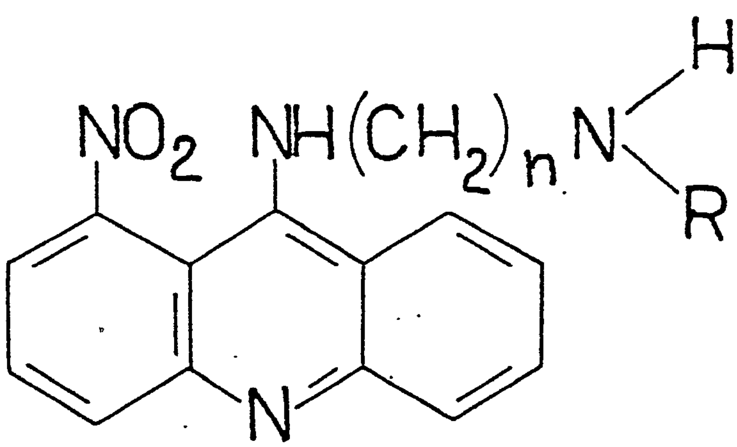

FORMULA 3

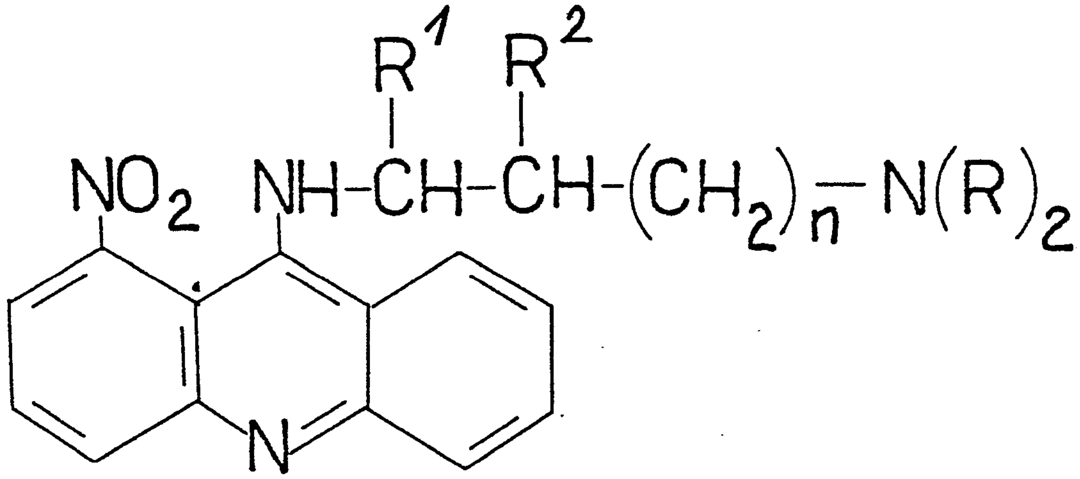

FORMULA 4